# EUROPÄISCHE PATENTSCHRIFT

(11) **EP 0 499 611 B1**
(45) Veröffentlichungstag und Bekanntmachung des Hinweises auf die Patenterteilung: **27.07.1994**
(21) Anmeldenummer: 90915996.4
(22) Anmeldetag: 03.11.1990
(51) Int. Cl.: C07C 209/54

(54) **VERFAHREN ZUR KONTINUIERLICHEN HERSTELLUNG VON 3,3'-DICHLOR-BENZIDIN-DIHIDROCHLORID**
PROCESS FOR THE CONTINUOUS PRODUCTION OF 3,3'-DICHLORO-BENZIDINE DIHYDROCHLORIDE
PROCEDE DE FABRICATION EN CONTINU DE DIHYDROCHLORURE DE 3,3'-DICHLORO-BENZIDINE

(30) Priorität: 09.11.1989 DE 3937320
(43) Veröffentlichungstag der Anmeldung: 26.08.1992
(73) Patentinhaber: HOECHST AKTIENGESELLSCHAFT, 65926 Frankfurt am Main (DE)
(72) Erfinder: HABIG, Kurt, D-6082 Mörfelden-Walldorf (DE); BAESSLER, Konrad, D-6230 Frankfurt am Main (DE); WARNING, Klaus, D-6239 Eppstein/Taunus (DE)
(86) Internationale Anmeldenummer: EP9001844
(87) Internationale Veröffentlichungsnummer: WO9107377

(56) Entgegenhaltungen:
- EP-A- 0 045 459
- EP-A- 0 046 185

## Beschreibung

Gegenstand der Erfindung ist ein Verfahren zur kontinuierlichen Herstellung von 3,3'-Dichlor-benzidindihydrochlorid aus 2,2'-Dichlor-hydrazobenzol durch Behandeln mit wäßriger Schwefelsäure in Gegenwart eines Alkalimetallsalzes eines Alkyl-polyglykolether-sulfats.

3,3'-Dichlor-benzidin besitzt die größte wirtschaftliche Bedeutung von allen bekannten Diphenylbasen. Sowohl die freie Base als auch besonders die mit Mineralsäuren gebildeten Salze stellen wertvolle Ausgangsmaterialien, beispielsweise für die Synthese von Farbstoffen, dar.

3,3'-Dichlor-benzidin wird üblicherweise aus 1-Chlor-2-nitrobenzol durch Reduktion und nachfolgende sogenannte Benzidin-Umlagerung des entstandenen 2,2'-Dichlorhydrazobenzols hergestellt. Die Umlagerung wird durch Mineralsäure katalysiert. Dazu wird das 2,2'-Dichlorhydrazobenzol zweckmäßig in einem aromatischen Lösemittel gelöst und dann wäßrige Mineralsäure hinzugefügt. Die Umlagerung findet dann in der wäßrigen Phase statt, wobei das entstandene 3,3'-Dichlor-benzidin-Salz die Bildung einer Suspension verursacht. Mit zunehmender Reaktionsdauer steigt die Viskosität der Suspension so sehr an, daß sie sich kaum noch rühren oder umpumpen läßt. Dies hat weiter zur Folge, daß der Stoffaustausch nur noch durch Diffusion erfolgen kann und für einen vollständigen Umsatz daher eine lange Reaktionsdauer notwendig wird. Unerwünschte Nebenreaktionen, wie z.B. Disproportionierungen, gewinnen dadurch an Bedeutung. Die Nebenprodukte vermindern die Ausbeute an 3,3'-Dichlor-benzidin und müssen überdies durch aufwendige Reinigungsoperationen entfernt werden.

Üblicherweise wird nur die für die Reaktion gerade notwendige Menge an Mineralsäure eingesetzt, um den Aufwand für die Aufarbeitung so gering wie möglich zu halten.

In der EP-OS 0 045 459 wird ein kontinuierliches Verfahren zur Herstellung von Diphenylbasen sowie den daraus mit Mineralsäuren gebildeten Salzen aus den entsprechenden N,N'-Diaryl-hydrazinen beschrieben. Die Lösung der Hydrazo-Verbindung in einem mit Wasser nicht mischbaren aromatischen Lösemittel wird dabei kontinuierlich mit einer solchen Menge an Mineralsäure versetzt, daß die Suspension förderbar bleibt. Als Lösemittel kommen bei diesem Verfahren alle unter den Reaktionsbedingungen gegenüber den an der Reaktion beteiligten Stoffen inerten flüssigen Aromaten mit einem ausreichenden Lösevermögen in Betracht. Technische Bedeutung besitzen dabei insbesondere Toluol, die isomeren Xylole oder das als "Solventnaphtha" bezeichnete handelsübliche Gemisch aus m-Xylol und Ethylbenzol.

Beim Verfahren der EP-OS 0 045 459 werden als Mineralsäuren 20 bis 80%ige wäßrige Schwefelsäure oder 10 bis 30%ige wäßrige Salzsäure verwendet. Diese Säuren werden in einem 10 bis 14fachen Überschuß über die zur Salzbildung notwendigen Menge zugesetzt. Die Reaktion wird bei einer Temperatur von 20 bis 50°C durchgeführt. Volumen und Ausgestaltung der Reaktionszone wird bestimmt durch die erforderliche Verweilzeit der Reaktionsmischung. Die Verweilzeit ihrerseits hängt ab von der Konstitution des N,N'-Diaryl-hydrazins und der Reaktionstemperatur. Bei einer Verweilzeit von 1 bis 3 Stunden hat sich eine Kaskade aus höchstens 5 Rührkesseln als vorteilhaft erwiesen.

Die Aufarbeitung des Reaktionsgutes erfolgt dann in bekannter Weise, z.B. durch Entfernung des aromatischen Lösemittels durch Destillation oder durch Abblasen mit Dampf und Abfiltration der wäßrigen Säure von dem ausgefallenen Salz der Diphenylbase, Rückführung des aromatischen Lösemittels und der auf den ursprünglichen Säuregehalt aufgestärkten wäßrigen Säure.

Als besonderer Vorzug des Verfahrens wird angegeben, daß mit einem Minimum an Operationen die Salze der Diphenylbase in hohen Ausbeuten und bei hohem Durchsatz erhalten werden, wobei die eingesetzten Hilfsstoffe vollständig in den Prozeß zurückgeführt werden können.

Zur Herstellung der reinen Salze der Diphenylbasen muß jedoch das zunächst anfallende rohe Salz entweder über die freie Base oder durch Umkristallisieren aus Salzsäure gereinigt werden.

Das Verfahren gemäß der EP-OS 0 045 459 hat zwar einen erheblichen Fortschritt gebracht, doch genügen die damit erreichbaren Raum-Zeit-Ausbeuten nicht mehr den heute gestellten Anforderungen. Bei der kontinuierlichen Umsetzung von 2,2'-Dichlor-hydrazobenzol mit wäßriger Schwefelsäure nach diesem Verfahren treten darüberhinaus unvorhersehbare Veränderungen in der Viskosität auf, die dazu führen, daß sich die Suspension kaum noch rühren oder umfüllen läßt. Aus der bei der Reaktion von 2,2'-Dichlorhydrazobenzol mit wäßriger Salzsäure gebildeten Suspension wurde bisher das Toluol im Dünnschichtverdampfer abdestilliert, wobei auch ein Teil der Salzsäure azeotrop abdestilliert wurde. Trotz des hohen apparativen Aufwandes zur Sicherstellung einer kontinuierlichen Verfahrensweise ließen sich Störungen im Verfahrensablauf, z.B. durch Schwierigkeiten beim Durchfluß durch den Verdampfer, nicht immer vermeiden.

Durch die vorliegende Erfindung wird nunmehr ein kontinuierliches Verfahren zur Herstellung von 3,3'-Dichlorbenzidin-dihydrochlorid zur Verfügung gestellt, bei welchem die vorstehend genannten Nachteile des bekannten Verfahrens nicht mehr auftreten.

Das erfindungsgemäße Verfahren zur kontinuierlichen Herstellung von 3,3'-Dichlor-benzidin-dihydrochlorid aus 2,2'-Dichlor-hydrazobenzol durch Behandeln mit wäßriger Schwefelsäure ist dadurch gekennzeichnet, daß man bei Temperaturen von 20 bis 50°C, vorzugsweise von etwa 36 bis etwa 40°C, in Gegenwart eines Alkalimetallsalzes, vorzugsweise des Natriumsalzes eines Alkyl(C₈-C₂₃)-polyglykolether-sulfats, das in einem nicht mit Wasser mischbaren aromatischen Lösemittel gelöste 2,2'-Dichlorhydrazobenzol kontinuierlich mit einer solchen Menge an 50 bis 80%iger, vorzugsweise etwa 60 bis etwa 65%iger wäßriger Schwefelsäure versetzt, daß die sich bildende Suspension förderbar bleibt, anschließend ebenfalls kontinuierlich die aus der Reaktionszone austretende Suspension mit Wasser verdünnt und anschließend auf eine Temperatur von 90 bis 95°C erhitzt bis eine Lösung erhalten wird, das aromatische Lösemittel von der heißen schwefelsauren wäßrigen Phase abtrennt und durch Zugabe von Salzsäure zu der verbleibenden schwefelsauren Lösung das 3,3'-Dichlor-benzidin-dihydrochlorid ausfällt und dieses filtriert.

Als nicht mit Wasser mischbare aromatische Lösemittel sind beispielsweise Toluol, die isomeren Xylole, o-Dichlorbenzol oder Lösungsmittelgemische, wie "Solventnaphtha" (Gemisch aus m-Xylol und Ethylbenzol) oder deren Mischungen geeignet.

Für die Umlagerungsreaktion werden zweckmäßigerweise 6 bis 7 Mol H₂SO₄ pro Mol 2,2'-Dichlor-hydrazobenzol eingesetzt.

Bei der Verdünnung der aus der Reaktionszone austretenden Suspension mit Wasser wird zweckmäßigerweise solange verdünnt, bis die wäßrige Phase noch etwa 40 Gew.-% H₂SO₄ enthält.

Die Zugabe der Salzsäure zur schwefelsauren wäßrigen Phase erfolgt kontinuierlich bei Temperaturen von 95 bis 110°C, wobei die 1,5 bis 2,5-fache Menge der zur Salzbildung erforderlichen Menge Salzsäure zugesetzt wird. Nach erfolgter Salzsäurezugabe wird die wäßrige Phase auf etwa 30 bis etwa 35°C abgekühlt und anschließend das ausgefallene 3,3'-Dichlor-benzidin-dihydrochlorid abfiltriert und dann mit Salzsäure gewaschen, bis die ablaufende Säure schwefelsäurefrei ist.

Das aromatische Lösemittel läßt sich problemlos abtrennen und kann nach erneuter Destillation wieder verwendet werden. Nach Zugabe der wäßriger Salzsäure, zweckmäßigerweise 10 bis 35 gewichtsprozentiger, vorzugsweise etwa 30 gewichtsprozentiger wäßriger Salzsäure, zu der heißen, 3,3'-Dichlor-benzidin-sulfat enthaltenden Lösung , wobei diese wäßrige Phase auf etwa 30 bis 35°C abgekühlt wird, scheidet sich reines kristallines 3,3'-Dichlorbenzidin-dihydrochlorid ab, das ohne Komplikationen abfiltriert und mit wenig Salzsäure frei von Schwefelsäure gewaschen werden kann. Die anfallende Mineralsäure läßt sich nach bekannten Verfahren, wie z.B. den von Bertrams oder von Schott beschriebenen, aufarbeiten und dann erneut verwenden. Das Verfahren kommt somit ohne Isolierung eines Zwischenproduktes aus und ist daher ökonomisch wie ökologisch vorteilhaft. Das vorliegende Verfahren kommt bei Einsatz einer etwa 62%igen wäßrigen Schwefelsäure mit einer theoretischen Verweilzeit von nur noch etwa 1 Stunde und einem nur noch etwa 6,1 fachen molaren Überschuß an Schwefelsäure, bezogen auf 2,2'-Dichlor-hydrazobenzol, aus.

Im Vergleich zum Stand der Technik, wie er in der EP-OS 0 045 459 zum Ausdruck kommt, bedeutet dies mehr als eine Halbierung der eingesetzten Menge an Schwefelsäure und eine wesentlich erhöhte Raum-Zeit-Ausbeute.

Beim Arbeiten (Umlagern) in Abwesenheit eines Alkalimetallsalzes eines Alkyl(C₈-C₂₃)-polyglykolethersulfats zeigt sich folgendes:
In einer Versuchsapparatur, bestehend aus 3 hintereinander geschalteten Rührkesseln, kann ein stationärer Betriebszustand während etwa 10 h ohne Schwierigkeiten aufrechterhalten werden. Bei einer Reaktionstemperatur von 36 bis 40°C sind im stationären Zustand beim Verlassen des ersten Rührkessels 88% und beim Verlassen des zweiten 97% des 2,2'-Dichlor-hydrazobenzols umgesetzt. Am Ausgang des dritten Rührkessels ist kein Ausgangsmaterial mehr nachweisbar, der Umsatz daher vollständig.

Nach 15 bis 20 h stationärem Betrieb verschlechtert sich unvorhersehbar die Fließfähigkeit des Reaktionsgemisches und es kommt zur vollständigen Verstopfung der Rohrleitungen, so daß der Versuch abgebrochen werden muß. Das mikroskopische Bild zeigt anstelle der bis dahin vorherrschenden stäbchenförmigen Kristalle nunmehr Feinstkristalle. Makroskopisch ist dieser Übergang an einer Aufhellung des Aspekts erkennbar.

Versucht man dann, die Dauer des anfänglich erreichten stationären Betriebszustandes durch Variation der Reaktionsparameter zu verlängern, indem man u.a. den Durchsatz um 25% erhöht, die Menge an Schwefelsäure um 10% vermehrt, die Konzentration an 2,2'-Dichlorhydrazobenzol von 24% auf 16% vermindert, die Rührfrequenz um 20% erhöht sowie einen anderen Rührertyp verwendet, so zeigt sich, daß sich durch keine dieser Maßnahmen die gewünschte Wirkung erzielen läßt. Auch durch den Zusatz von Emulgatoren auf der Basis von Alkylpolyglykolethern, organischen Phosporsäureestern oder Alkylbenzolsulfonaten läßt sich der beschriebene Übergang zu den störenden Feinstkristallen nicht verhindern.

Arbeitet man jedoch in Gegenwart eines Alkalimetallsalzes eines Alkyl(C₈-C₂₃)-polyglykolether-sulfats, so bleibt überraschenderweise der stationäre Betriebszustand im Rahmen der kontinuierlichen Verfahrensdurchführung erhalten.

Die Alkalimetallsalze eines Alkyl(C₈-C₂₃)-polyglykolethersulfats, vorzugsweise deren Natriumsalze, besonders bevorzugt das C₁₂H₂₅-O-(C₂H₄-O)₁₋₄-SO₃Na, sind hierbei in einer Konzentration von etwa 0,04 bis etwa 1,0 Gew.-%, vorzugsweise etwa 0,1 bis etwa 0,4 Gew.-%, bezogen auf das Gewicht der Reaktionsmischung, wirksam. Sie können in Form wäßriger Lösungen der zuzusetzenden Schwefelsäure beigegeben oder getrennt dem Inhalt des ersten Rührkessels zudosiert werden. Durch das Arbeiten (Umlagern) in Anwesenheit eines Alkalimetallsalzes eines Alkyl(C₈-C₂₃)-polyglykolethersulfats läßt sich ein stationärer Betriebszustand im Rahmen des Kontinueverfahrens auf Dauer erhalten.

### Beispiel

### Reaktionsapparatur:

Kaskade, bestehend aus 3 Rührkesseln gleichen Volumens, jeweils mit Kühlmantel sowie Schleifen- oder Impellerrührer (300 Umdrehungen pro min.) versehen. Gesamt-Reaktionsvolumen 2,8 l. Alle Rührkessel sind durch Tauchrohre miteinander verbunden. Die Ausgangskomponenten werden in den ersten Rührkessel unter die Flüssigkeitsoberfläche kontinuierlich eindosiert.
Theoretische Verweilzeit: 60 min.
Durchsatz pro Stunde:
407 g 2,2'-Dichlorhydazobenzol 1660 g
1253 g Solventnaphtha (1750 ml)
1550 g (1000 ml) 62%ige wäßrige Schwefelsäure
50 g (50 ml) 3%ige wäßrige Lösung des c₁₂H₂₅-O-(C₂H₄O-)₂-SO₃Na
Reaktionstemperatur: 36 bis 40°C
In Abwesenheit des Alkyl-polyglykolether-sulfats ändert sich spätestens nach 15 bis 20 h Dauerbetrieb die Kristallstruktur des 3,3'-Dichlor-benzidin-sulfats, was sich durch totale Verstopfung aller Rohrleitungen äußert. Dagegen wird in Gegenwart eines Alkyl(C₈-C₂₃)-polyglykolether-sulfats die grobkristalline Modifikation stabilisiert, wodurch ein ungestörter kontinuierlicher Betrieb sichergestellt wird.

Die Umwandlung des 3,3'-Dichlor-benzidin-sulfats in 3,3'-Dichlor-benzidin-dihydrochlorid wird in den kontinuierlichen Betrieb einbezogen. Dazu wird die aus dem dritten Rührkessel ablaufende Suspension mit 840 g Wasser/h versetzt und dann auf 90 bis 95°C erhitzt, wobei Lösung eintritt. Die aromatische Phase läßt sich dann problemlos abtrennen und wird nach Destillation wiederverwendet. Nach Zugabe von ca. 30%iger wäßriger Salzsäure zu der verbleibenden heißen schwefelsauren Lösung (Temperatur 95 - 110°C) fällt beim Abkühlen reines kristallines 3,3'-Dichlor-benzidin-dihydrochlorid aus, das abfiltriert und mit Salzsäure schwefelsäurefrei gewaschen wird.

Die Ausbeute an 3,3'-Dichlor-benzidin-dihydrochlorid beträgt mindestens 80% d. Th., bezogen auf 1-Chlor-2-nitrobenzol bzw. 88 bis 89% d. Th., bezogen auf 2,2'-Dichlorhydrazobenzol.
Restfeuchte: 6 bis 8 Gew.-%.
Diazowert: 77,8%, bezogen auf die freie Base (getrocknetes Dichlorhydrat).

Die anfallende überschüssige Mineralsäure kann nach bekannten Verfahren, beispielsweise nach Bertrams bzw. Schott, wiederaufgearbeitet und erneut eingesetzt werden.

Verwendet man anstelle von 1253 g Solventnaphtha die gleiche Gewichtsmenge an o-, m-, p-Xylol oder o-Dichlorbenzol und arbeitet im übrigen wie im Beispiel angegeben, so gelangt man praktisch zum gleichen Ergebnis.

## Patentansprüche

1. Verfahren zur kontinuierlichen Herstellung von 3,3'-Dichlor-benzidin-dihydrochlorid aus 2,2'-Dichlorhydrazobenzol durch Behandeln mit wäßriger Schwefelsäure, dadurch gekennzeichnet, daß man bei Temperaturen von 20 bis 50°C in Gegenwart eines Alkalimetallsalzes eines Alkyl(C₈-C₂₃)-polyglykolether-sulfats das in einem nicht mit Wasser mischbaren aromatischen Lösemittel gelöste 2,2'-Dichlorhydrazobenzol kontinuierlich mit einer solchen Menge 50 bis 80%iger wäßriger Schwefelsäure versetzt, daß die sich bildende Suspension förderbar bleibt, anschließend ebenfalls kontinuierlich die aus der Reaktionszone austretende Suspension mit Wasser verdünnt und anschließend auf eine Temperatur von 90 bis 95°C erhitzt, bis eine Lösung erhalten wird, das aromatische Lösemittel von der heißen schwefelsauren wäßrigen Phase abtrennt und durch Zugabe von Salzsäure zu der verbleibenden schwefelsauren Lösung das 3,3'-Dichlor-benzidin-dihydrochlorid ausfällt und dieses abfiltriert.

2. Verfahren nach Anspruch 1, dadurch gekennzeichnet, daß man in Gegenwart des Natriumsalzes von Alkyl(C₈-C₂₃)--O-(C₂H₄-O)₁₋₄-SO₃H arbeitet.

3. Verfahren nach mindestens einem der Ansprüche 1 und 2, dadurch gekennzeichnet, daß man in Gegenwart des Natriumsalzes von (C₁₂H₂₅)-O-(C₂H₄O)₂-SO₃H arbeitet.

4. Verfahren nach mindestens einem der Ansprüche 1 bis 3, dadurch gekennzeichnet, daß man mit etwa 60 bis etwa 65%iger wäßriger Schwefelsäure arbeitet.

5. Verfahren nach mindestens einem der Ansprüche 1 bis 4, dadurch gekennzeichnet, daß man 6 bis 7 Mol Schwefelsäure pro Mol 2,2'-Dichlor-hydrazobenzol einsetzt.

6. Verfahren nach mindestens einem der Ansprüche 1 bis 5, dadurch gekennzeichnet, daß man die Behandlung mit der wäßrigen Schwefelsäure bei einer Temperatur von etwa 36 bis etwa 40°C durchführt.

7. Verfahren nach mindestens einem der Ansprüche 1 bis 6, dadurch gekennzeichnet, daß man als aromatisches Lösemittel Toluol, ein isomeres Xylol, o-Dichlorbenzol oder Solventnaphtha oder deren Mischungen verwendet.

8. Verfahren nach mindestens einem der Ansprüche 1 bis 7, dadurch gekennzeichnet, daß man die aus der Reaktionszone austretende Suspension mit Wasser verdünnt, bis die wäßrige Phase etwa 40 Gew.-% Schwefelsäure enthält und anschließend auf 90 bis 95°C erhitzt, bis eine Lösung erhalten wird.

9. Verfahren nach mindestens einem der Ansprüche 1 bis 8, dadurch gekennzeichnet, daß die Salzsäure kontinuierlich bei einer Temperatur von 95 bis 110°C der schwefelsauren wäßrigen Phase zugesetzt wird.

10. Verfahren nach mindestens einem der Ansprüche 1 bis 9, dadurch gekennzeichnet, daß die 1,5 bis 2,5-fache Menge der zur Salzbildung erforderlichen Menge Salzsäure der schwefelsauren wäßrigen Phase zugesetzt wird.

11. Verfahren nach mindestens einem der Ansprüche 1 bis 10, dadurch gekennzeichnet, daß eine 10 bis 35 gew.-%ige wäßrige Salzsäure der schwefelsauren wäßrigen Phase zugesetzt wird.

12. Verfahren nach mindestens einem der Ansprüche 1 bis 11, dadurch gekennzeichnet, daß die wäßrige Phase nach Zugabe der Salzsäure auf etwa 30 bis etwa 35°C abgekühlt und anschließend das ausgefallene 3,3'-Dichlor-benzidindihydrochlorid abfiltriert und nachfolgend mit Salzsäure gewaschen wird, bis die ablaufende Säure schwefelsäurefrei ist.

13. Verfahren nach mindestens einem der Ansprüche 1 bis 12, dadurch gekennzeichnet, daß man in Gegenwart einer Konzentration von etwa 0,04 bis etwa 1,0 Gew.-% des Alkalimetallsalzes des Alkyl(C₈-C₂₃)-polyglykolethersulfats, bezogen auf das Gewicht der Reaktionsmischung, arbeitet.

14. Verfahren nach mindestens einem der Ansprüche 1 bis 13, dadurch gekennzeichnet, daß man in Gegenwart einer Konzentration von etwa 0,1 bis etwa 0,4 Gew.-% des Alkalimetallsalzes des Alkyl(C₈-C₂₃)-polyglykolethersulfats, bezogen auf das Gewicht der Reaktionsmischung, arbeitet.

15. Verfahren nach mindestens einem der Ansprüche 1 bis 14, dadurch gekennzeichnet, daß die überschüssige Säure nach ihrer Abtrennung wiederaufgearbeitet und erneut verwendet wird.

## Claims

1. A process for the continuous preparation of 3,3'-dichlorobenzidine dihydrochloride from 2,2'-dichlorohydrazobenzene by treatment with aqueous sulfuric acid, which comprises treating the 2,2'-dichlorohydrazobenzene, dissolved in a water-immiscible aromatic solvent, continuously at temperatures of from 20 to 50°C in the presence of an alkali metal salt of an alkyl(C₈-C₂₃)polyglycol ether sulfate, with such an amount of from 50 to 80 % strength aqueous sulfuric acid that the suspension formed remains conveyable, subsequently diluting the suspension emerging from the reaction zone with water, again continuously, and subsequently heating the suspension to a temperature of from 90 to 95°C until a solution is obtained, separating off the aromatic solvent from the hot, sulfuric acid aqueous phase, and precipitating the 3,3'-dichlorobenzidine dihydrochloride by adding hydrochloric acid to the sulfuric acid solution which remains, and filtering off this product.

2. The process as claimed in claim 1, carried out in the presence of the sodium salt of alkyl(C₈-C₂₃)-O-(C₂H₄-O)₁₋₄-SO₃H.

3. The process as claimed in at least one of claims 1 and 2, carried out in the presence of the sodium salt of (C₁₂H₂₅)-O-(C₂H₄O)₂-SO₃H.

4. The process as claimed in at least one of claims 1 to 3, carried out using from about 60 to about 65 % strength aqueous sulfuric acid.

5. The process as claimed in at least one of claims 1 to 4, wherein from 6 to 7 mol of sulfuric acid are employed per mol of 2,2'-dichlorohydrazobenzene.

6. The process as claimed in at least one of claims 1 to 5, wherein the treatment with aqueous sulfuric acid is carried out at a temperature from about 36 to about 40°C.

7. The process as claimed in at least one of claims 1 to 6, wherein the aromatic solvent used is toluene, an isomeric xylene, o-dichlorobenzene or Solvent Naphtha, or a mixture thereof.

8. The process as claimed in at least one of claims 1 to 7, wherein the suspension emerging from the reaction zone is diluted with water until the aqueous phase contains about 40 % by weight of sulfuric acid and is subsequently heated to from 90 to 95°C until a solution is obtained.

9. The process as claimed in at least one of claims 1 to 8, wherein the hydrochloric acid is added continuously to the sulfuric acid aqueous phase at a temperature of from 95 to 110°C.

10. The process as claimed in at least one of claims 1 to 9, wherein from 1.5 to 2.5 times the amount of hydrochloric acid necessary for salt formation is added to the sulfuric acid aqueous phase.

11. The process as claimed in at least one of claims 1 to 10, wherein from 10 to 35 % strength by weight aqueous hydrochloric acid is added to the sulfuric acid aqueous phase.

12. The process as claimed in at least one of claims 1 to 11, wherein the aqueous phase is cooled to from about 30 to about 35°C after the hydrochloric acid has been added, and the precipitated 3,3'-dichlorobenzidine dihydrochloride is subsequently filtered off and then washed with hydrochloric acid until the acid washings are free from sulfuric acid.

13. The process as claimed in at least one of claims 1 to 12, carried out in the presence of a concentration of from about 0.04 to about 1.0 % by weight of the alkali metal salt of the alkyl(C₈-C₂₃)polyglycol ether sulfate, based on the weight of the reaction mixture.

14. The process as claimed in at least one of claims 1 to 13, carried out in the presence of a concentration of from about 0.1 to about 0.4 % by weight of the alkali metal salt of the alkyl(C₈-C₂₃)polyglycol ether sulfate, based on the weight of the reaction mixture.

15. The process as claimed in at least one of claims 1 to 14, wherein the excess acid is worked up again after separation and is re-used.

## Revendications

1. Procédé de préparation en continu de dichlorhydrate de 3,3'-dichlorobenzidine à partir de 2,2'-dichlorohydrazobenzène par traitement avec de l'acide sulfurique aqueux, caractérisé en ce que, à des températures de 20 à 50°C, en présence d'un sel de métal alcalin d'un (alkyl en C₈-C₂₃)-polyglycoléthersulfate, on ajoute en continu au 2,2'-dichlorohydrazobenzène dissous dans un solvant aromatique non miscible à l'eau une quantité d'acide sulfurique aqueux à 50 à 80 % telle que la suspension qui se forme puisse toujours être transvasée, on dilue ensuite, également en continu, la suspension sortant de la zone de réaction avec de l'eau, puis on la chauffe à une température de 90 à 95°C jusqu'à ce que l'on obtienne une solution, on sépare le solvant aromatique de la phase aqueuse chaude contenant de l'acide sulfurique, et on précipite le dichlorhydrate de 3,3'-dichlorobenzidine en ajoutant de l'acide chlorhydrique à la solution contenant de l'acide sulfurique restante, et on le filtre.

2. Procédé selon la revendication 1, caractérisé en ce que l'on travaille en présence du sel de sodium d'un (alkyl en C₈-C₂₃)-O-(C₂H₄-O)₁₋₄-SO₃H.

3. Procédé selon au moins l'une des revendications 1 et 2, caractérisé en ce que l'on travaille en présence du sel de sodium de C₁₂H₂₅-O-(C₂H₄-O)₂-SO₃H.

4. Procédé selon au moins l'une des revendications 1 à 3, caractérisé en ce que l'on travaille avec de l'acide sulfurique aqueux à une concentration d'environ 60 à environ 65 %.

5. Procédé selon au moins l'une des revendications 1 à 4, caractérisé en ce que l'on utilise 6 à 7 moles d'acide sulfurique par mode de 2,2'-dichloro-hydrazobenzène.

6. Procédé selon au moins l'une des revendications 1 à 5, caractérisé en ce que l'on effectue le traitement avec l'acide sulfurique aqueux à une température d'environ 36 à environ 40°C.

7. Procédé selon au moins l'une des revendications 1 à 6, caractérisé en ce que l'on utilise comme solvant aromatique du toluène, un isomère de xylène, de l'odichlorobenzène ou du solvant naphta, ou leurs mélanges.

8. Procédé selon au moins l'une des revendications 1 à 7, caractérisé en ce que l'on dilue la suspension sortant de la zone de réaction avec de l'eau jusqu'à ce que la phase aqueuse contienne environ 40 % en masse d'acide sulfurique, puis on la chauffe à une température de 90 à 95°C jusqu'à ce que l'on obtienne une solution.

9. Procédé selon au moins l'une des revendications 1 à 8, caractérisé en ce que l'on ajoute en continu l'acide chlorhydrique à la phase aqueuse contenant de l'acide sulfurique à une température de 95 à 110°C.

10. Procédé selon au moins l'une des revendications 1 à 9, caractérisé en ce que l'on ajoute à la phase aqueuse contenant de l'acide sulfurique une quantité d'acide chlorhydrique égale à 1,5 à 2,5 fois la quantité nécessaire à la formation du sel.

11. Procédé selon au moins l'une des revendications 1 à 10, caractérisé en ce que l'on ajoute à la phase aqueuse contenant de l'acide sulfurique un acide chlorhydrique aqueux à une concentration de 10 à 35 % en masse.

12. Procédé selon au moins l'une des revendications 1 à 11, caractérisé en ce que l'on refroidit la phase aqueuse après l'addition de l'acide chlorhydrique à une température d'environ 30 à environ 35°C, on filtre ensuite le dichlorhydrate de 3,3'-dichlorobenzidine précipité, puis on le lave avec de l'acide chlorhydrique jusqu'à ce que l'acide qui s'écoule ne contienne plus d'acide sulfurique.

13. Procédé selon au moins l'une des revendications 1 à 12, caractérisé en ce que l'on travaille en présence d'une concentration d'environ 0,04 à environ 1,0 % en masse, par rapport à la masse du mélange réactionnel, du sel alcalin de l'(alkyl en C₈-C₂₃)-polyglycoléthersulfate.

14. Procédé selon au moins l'une des revendications 1 à 13, caractérisé en ce que l'on travaille en présence d'une concentration d'environ 0,1 à environ 0,4 % en masse, par rapport à la masse du mélange réactionnel, du sel alcalin de l'(alkyl en C₈-C₂₃)-polyglycoléthersulfate.

15. Procédé selon au moins l'une des revendications 1 à 14, caractérisé en ce que l'on retraite et on réutilise l'acide en excès après l'avoir séparé.
